(19) Europäisches Patentamt

European Patent Office

Office européen des brevets

(11) **EP 1 067 198 A1**

(12) **EUROPÄISCHE PATENTANMELDUNG**

(43) Veröffentlichungstag:
**10.01.2001 Patentblatt 2001/02**

(51) Int Cl.[7]: **C12Q 1/00**, C12M 1/34

(21) Anmeldenummer: 00710008.4

(22) Anmeldetag: **29.06.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **01.07.1999 DE 19930496**

(71) Anmelder: **Degussa-Hüls Aktiengesellschaft**
**60287 Frankfurt am Main (DE)**

(72) Erfinder:
- **Bommarius, Andreas Dr.**
  **60596 Frankfurt (DE)**

- **Estler, Manfred Dr.**
  **63584 Gründau (DE)**
- **Kluge, Andreas Dr.**
  **63179 Obertshausen (DE)**
- **Werner, Helge**
  **63486 Bruchköbel (DE)**
- **Vollmer, Helga Dr.**
  **63457 Hanau (DE)**
- **Bock, Hans-Georg Prof. Dr.**
  **69117 Heidelberg (DE)**
- **Schlöder, Johannes P. Dr.**
  **69181 Leimen (DE)**
- **Kostina, Ekaterina Dr.**
  **69117 Heidelberg (DE)**

(54) **Verfahren zur Bestimmung der Prozessstabilität von Enzymen**

(57) Die Erfindung betrifft ein mehrstufiges Verfahren zur Bestimmung der Prozeßstabilität von Enzymen durch Aufnahme von Temperatur-Zeit-Profilen der zu prüfenden Reaktion in einem kontinuierlich betriebenen, instationären Differential-, Kreislauf- oder Rührreaktor und Ermittlung der charakteristischen Aktivitäts- und Stabilitätsparameter durch numerische Methoden in bevorzugt vier Schritten.

EP 1 067 198 A1

## Beschreibung

Die Erfindung betrifft ein Verfahren zur schnellen Bestimmung der Prozeßstabilität von Enzymen.

Diese sind als Biokatalysatoren seit langem bekannt und werden aufgrund ihrer hohen Aktivität und Selektivität in Analyse und Synthese intensiv untersucht.

Dem Einsatz im industriellen Maßstab stand in der Vergangenheit in vielen Fällen der große Aufwand zur Beurteilung des Langzeitverhaltens derartiger Enzyme im Wege.

Die empirische Ermittlung der Stabilitätsparameter erfolgt in langwierigen Laborversuchen, wobei für eine jeweils konstant gehaltene Temperatur die Halbwertszeit des Katalysators bestimmt wird. Das bedeutet, daß bei konstanten Reaktionsbedingungen die Zeit aufgenommen wird, in der die Katalysatoraktivität von dem Anfangswert auf die Hälfte bzw. einen signifikant meßbaren Teil dieses Wertes abnimmt (M. N. Gupta: "Thermostability of Enzymes", Springer Verlag & Norsa Publ. House, Berlin, New York, 1993; Sadana: "Biocatalysis- Fundamentals of Enzyme Deactivation Kinetics", Prentice Hall, New Jersey 1991).

Dieses Verfahren wird für verschiedene Temperaturen wiederholt. Dabei wird die Temperatur des Katalysators in diskreten Schritten langsam erhöht, bis die Temperatur so hoch ist, daß es zu einer sofortigen und offensichtlichen Zerstörung der Katalysatoren kommt.

Ein besonderer Nachteil dieses Verfahrens liegt in dem großen Zeitaufwand für die Versuche. Dies ist vor allem dann der Fall, wenn der Katalysator bei verfahrensgemäß niedrigen Reaktionstemperaturen eine große Halbwertszeit besitzt. Darüber hinaus ist die Bestimmung des Temperaturoptimums durch die Temperaturerhöhung in diskreten Schritten nicht notwendigerweise exakt. Der Fehler nimmt mit steigender Schrittweite stark zu. Die Desaktivierung durch thermisch verursachte Alterung ist ebenfalls temperaturabhängig und nimmt mit steigender Temperatur zu.

Eine optimale Betriebstemperatur Temperaturpunkt kann bisher nur durch Ermittlung der Desaktivierungskurven für jede einzelne Reaktionstemperatur gefunden werden. Dies bedeutet, daß theoretisch unendlich viele Versuche durchgeführt werden müssen, um den gewünschten Arbeitspunkt im Sinne erwünschter Raum-Zeit-Ausbeute zu finden.

Ein Ansatz zur schnellen Ermittlung der relevanten Werte wird in der DE 195 46 192 A1 (ebenso M. Boy et al., Chem. Eng. Technol. 21, (1998), 570 - 575) beschrieben.

In einem instationär betriebenen Reaktor werden Temperatur-Zeit-Profile ermittelt, und aus den Meßkurven mit bekannten numerischen Methoden sechs Aktivitäts- und Stabilitätsparameter ermittelt. Es wird bei diesem Verfahren an keiner Stelle berücksichtigt, daß durch eine nahezu beliebige Anzahl von möglichen Kombinationen dieser Parameter eine nahezu identische Anpassung der Meßdaten liefern. Eine Angabe der Schätzgenauigkeit für die Modellparameter erfolgt ebenfalls nicht.

Das führt dazu, daß der Fachmann die gewünschten Parameter nach dieser Methode nicht signifikat bestimmen kann.

Wenn eine große Anzahl von Wertepaaren für Enthalpie und Entropie (Arrhenius-Gleichung) ermittelt werden können, bleibt die Auswahl des richtigen dem Zufall überlassen.

Bei den bevorzugt bearbeiteten Hydrolasen mißt man den Reaktionsfortschritt bei konstantem pH durch Verbrauch von Lauge bei der Hydrolyse. Für die Auswertung steht der zeitliche Verbrauch an Lauge, die zur Neutralisation der auf Grund der Enzymaktivität entstehenden Säure benötigt wird, zur Verfügung. Eine Hauptschwierigkeit besteht hierbei, daß über ein und dasselbe Meßsignal mehrere parallel verlaufende Reaktionsmechanismen gleichzeitig erfaßt werden. Eine Unterscheidung der Mechanismen (Aktivierung, Entfaltung und Desaktivierung) kann nur dadurch erfolgen, daß in bestimmten Temperaturbereichen jeweils ein Mechanismus dominierend ist.

Der am Ende der Reaktionskette stehende Schritt der irreversiblen Katalysatordesaktivierung stellt dabei den am schwierigsten zu identifizierenden Abschnitt dar. Erschwerend kommt an dieser Stelle hinzu, daß eine starke Abhängigkeit zwischen den beiden diesen irreversiblen Vorgang beschreibenden Modellparametern besteht. Dies bedeutet, daß im Prinzip beliebig viele Parameterkombinationen existieren, die das gemessene Meßsignal mit nahezu gleicher Genauigkeit beschreiben. Es ist daher möglich, daß Parameterwerte berechnet werden, die einerseits in einem physikalisch nicht interpretierbaren Wertebereich liegen und andererseits eine gute Anpassung an das Meßsignal liefern. Dieses Problem ist bei den aus dem Stand der Technik bekannten Arbeiten nicht berücksichtigt worden. A priori kann nicht bewertet werden, welches Temperaturprofil eine gute Parameterschätzung erlaubt: die Varianz der Schätzung hängt von den durchgeführten Experimenten ab. Ein rein lineares Profil ist aber ungeeignet, da die Berechnung der Startwerte schon stark fehlerhaft ist. Damit ist die Konvergenz eines nachgeschalteten Optimierverfahrens nicht gesichert. Auch eine Betrachtung der Fehlerfortpflanzung von Parameterschätzfehlern im Hinblick auf das berechnete Langzeitstabilitätsverhalten findet nicht statt.

Das Problem der zuverlässigen Vorhersage des Langzeitverhaltens von Enzymkatalysatoren durch verhältnismäßig schnell zu ermittelnde Daten bleibt daher im Stand der Technik ungelöst.

Aufgabe der Erfindung ist es, ein Verfahren bereitzustellen, mit dessen Hilfe die zur Bestimmung der Prozeßstabilität notwendigen Enzymkenngrößen mit hinreichender Genauigkeit schnell bestimmt werden können.

Den zu ermittelnden Meßdaten liegt folgendes Modell zugrunde:

Im Gegensatz zur Situation bei chemischen homogenen und heterogenen Katalysatoren ist die thermische Desaktivierung von Enzymen gut in wenigen Schritten zu beschreiben:

$$N \iff U$$
$$\searrow \quad \swarrow$$
$$D$$

N =  natives Enzym, aktiv, meßbar
U =  enfaltetes (unfolded), Enzym nicht meßbar
D =  desaktiviertes Enzym, nicht meßbar

Daraus ergibt sich die als für das erfindungsgemäße Verfahren die notwendige Bestimmung der folgenden Kenngrößen:

1. Desaktivierungsenthalpie:
Bei der Aktivierung kann man die Abhängigkeit der Reaktionsgeschwindigkeit r von der absoluten Temperatur mit der Arrhenius-Gleichung beschreiben:

$$r = A \cdot [E]_0 \exp(E_a/RT)$$

Die Aktivierungsenthalpie $E_a$ kann in einer halblogarithmischen Darstellung von ln r über (1/T) aus der Steigung der resultierenden Gerade berechnet werden. Hier wird die Aktivierungsenthalpie $E_a$ als $\Delta h_E^*$ und die Entropie (der präexponentielle Faktor) als A bezeichnet.
Analog läßt sich eine solche Arrhenius-Gleichung auch für die beiden Desaktivierungsprozesse $N \rightarrow D$ und $U \rightarrow D$ aufstellen, da beide Prozesse als nach erster Ordnung verlaufend angenommen werden. Wenn N bzw. U allgemein als Spezies J bezeichnet werden, gilt:

$$k_{des,J} = k_{des,J}^0 \exp(E_{a,des,J}/RT),$$

sodaß aus einer Auftragung von ln $k_{des,J}$ über 1/T eine Gerade der Steigung $E_{a,des,J}$ resultiert. Für diese Geraden werden als Desaktivierungsenthalpien $E_{a,des,J}$ als $\Delta h_{d,N}^*$ (von N aus) und als $\Delta h_{d,u}$ (von U aus) bezeichnet. Die dazugehörigen Konstanten $k_{des,J}^0$ (also die Entropien) werden als $k_{d,n}^0$ und $k_{d,U}^0$ bezeichnet.

2. Schmelzpunkt:
Am Schmelzpunkt $T_m$ geht N reversibel in U über. Unterhalb von $T_m$ dominiert der Prozeß $N \rightarrow D$ mit der dazugehörigen Desaktivierungsenthalpie und Konstante, oberhalb von $T_m$ der Prozeß $U \rightarrow D$ mit den entsprechenden Parametern. Der Schmelzvorgang ist mit einer Enthalpie $\Delta h_U^0$ und einer Entropie $\Delta S_U^0$ verbunden. Es gilt: $T_m = \Delta h_U^0 / \Delta S_U^0$.

3. Die zu bestimmenden Parameter:
Aus den Punkten 1 und 2 ergibt sich, daß insgesamt acht Konstanten zu bestimmen sind: zwei Aktivierungsparameter ($\Delta h_E^*$ und A), zwei Entfaltungsparameter ($\Delta h_U^0$ und $\Delta s_U^0$) und vier Desaktivierungsparameter ($\Delta h_{d,N}^*$ und $k_{d,N}^0$ (von N aus) und $\Delta h_{d,U}^*$ und $k_{d,U}^0$ (von U aus)) :

4. Das Langzeitverhalten wird durch Enzymgrößen wie Total Turnover number (TTN) und die Halbwertszeit charaktierisiert.

Gegenstand der Erfindung ist ein vierstufiges Verfahren zur Bestimmung der Prozeßstabilität von Enzymen durch Aufnahme von Temperatur-Zeit-Profilen der zu prüfenden Reaktion in einem kontinuierlich betriebenen, instationären Differential-, Kreislauf- oder Rührreaktor und Ermittlung der charakteristischen Aktivitäts- und Stabilitätsparameter durch numerische Methoden in vier Schritten.

Folgende Schritte werden im einzelnen durchgeführt:

1. Experimentelle Ermittlung von acht Startparametern und Berechnung der Arrhenius-Kurve der Desaktivierung (ln $k_{des,J}$ über 1/T) mit den so erhaltenen Startwerten,

2. Mathematische Berechnung von speziellen Temperatur-Zeit-Profilen als Vorgabe für neue Experimente zur Aufnahme von Umsatz-Zeit-Kurven.

3. Auf Basis dieser neuen experimentellen Daten erfolgt die Verbesserung der Parameterschätzung mit Hilfe bekannter numerischer Optimierungsverfahren und eine Angabe der Schätzgüte für diese Parameter.

4. Errechnung der prozeßrelevanten Parameter:

   i) total turnover (TTN),
   ii) Halbwertszeit ($\tau_{1/2}$) und
   iii) (optional): optimale Temperatur $T_{opt}$ bei bestimmter Mindestraumzeitausbeute und Angabe der Schätzgüte.

Bei Bedarf (z.B. bei noch nicht vollständig erfüllten Genauigkeitsanforderungen) können die Schritte 2 bis 4 mehrfach iterativ durchlaufen werden.

Nachfolgend werden die einzelnen Schritte dieses vierstufigen Verfahrens genauer erläutert.

Die praktische Durchführung der Ermittlung geeigneter Startwerte für die Parameter erfolgt so, daß man im ersten Schritt in einer an sich bekannten Apparatur unter Einsatz bekannter Meßverfahren

1.1 in einem Temperaturbereich von ca. 20°C bis 90°C den Reaktionsverlauf mißt und daraus

1.1.1 eine erste Schätzung für den Schmelzpunkt $T_m$ und

1.1.2 die Aktivierungsparameter $\Delta h_E^*$ und A bestimmt,

1.2 die Desaktivierungs- bzw. die Halbwertszeit des Enzyms bei $\geq$ sechs Temperaturen ermittelt,

1.2.1 die zugehörigen Enthalpien $\Delta h_{d,U}^*$/ oberhalb und $\Delta h_{d,N}^*$ unterhalb $T_m$ aus dem zugehörigen Arrhenius-Diagramm ebenso

1.2.2 die Desaktivierungsentropien des nativen Zustands $k_{d,N}^0$ und des entfalteten Zustands $k_{d,U}^0$ aus den Halbwertszeiten $\tau_{1/2}$ der Desaktivierungskurven unterhalb ($k_{d,N}^0$) und oberhalb ($k_{d,N}^0$) ebenfalls aus dem Arrhenius-Diagramm, und

1.2.3 $\Delta h_U^0$ und $\Delta s_U^0$ durch Extrapolation der Geraden aus dem Arrhenius-Diagramm (2.1.1) und Bestimmung des Schnittpunktes. Der x-Wert entspricht $1/T_m$. $\Delta S_U^0$ wird aus dem Residuum der gesamten Reaktionsrate abzüglich des Aktivierungsanteiles in einem angemessenen Temperaturbereich um den Schmelzpunkt $T_m$ ermittelt. Die Bestimmung der Parameter erfolgt über ein Arrhenius-Diagramm für das Residuum. Dabei gilt $\Delta h_U^0 = T_m \cdot \Delta s_U^0$.
   Alternativ oder zusätzlich können zur Bestimmung der Startwerte gemäß den Schritten 1.1 und 1.2 auch gängige Parameterschätzverfahren eingesetzt werden.

Damit sind alle Startwerte der acht Parameter ermittelt.

Es wurde nun gefunden, daß es nicht ausreicht, diese oder auf anderem Wege ermittelte Startwerte als Basis für zufriedenstellende Parameteroptimierungen z. B. nach der sequentiellen quadratischen Programmierung (SQP) oder auf Basis eines Randwertproblemansatzes einzusetzen, wie es im Prinzip der in der DE-OS 195 46 192 beschriebenen Methode entspricht. Dabei ist zusätzlich zu berücksichtigen, daß nach dem Stand der Technik ein anderer Aktivierungs- / Desaktivierungsmechanismus vorausgesetzt wird.

Wie die Erfahrung weiter zeigt, reicht es nicht aus, eine Zahl von Startwerten für die Parameterschätzung durch eine Optimierung nach der Methode der kleinsten Fehlerquadrate an ein beliebiges Temperatur-Zeit-Profil oder ein Temperatur-Zeit-Profil mit linearem Temperatur-Anstieg anzupassen.

Vielmehr werden erfindungsgemäss Temperatur-Zeit-Profile in Schritt 2 derart bestimmt, dass sie möglichst viele Start-Informationen über die relevanten Parameter liefern. Ziel der Bestimmung dieser Profile ist es, die Minimierung der Bereiche der ermittelten Parameterwerte einzugrenzen.

Auf Basis der gemäß Punkt 1. ermittelten Startwerte für acht Parameter wird entweder empirisch oder auf

EP 1 067 198 A1

Basis eines allgemein bekannten Algorithmus zum Optimum Experimental Design (I. Bauer, S. Körkel, H.G. Bock, J. P. Schlöder: "Optimale Versuchsplanung für dynamische Systeme aus der chemischen Reaktionskinetik", In: Optimale Versuchsplanung für nichtlineare Prozesse, Informationstag am 14.10.1998 in Frankfurt am Main, DECHEMA e.V., pp. 1-31, 1998) eine definierte Anzahl von Temperatur-Zeit-Profilen errechnet, die notwendig sind, um die gewünschte Genauigkeit für die Modellparameter zu erreichen.

Nach Durchführung der entsprechenden Experimente erfolgt im Schritt 3 eine Parameterschätzung durch Optimierung im Sinne der kleinsten Fehlerquadrate (SQP oder Randwertproblemansatz).

Aus den auf diesem Wege erhaltenen Meßkurven können im Schritt 4 aussagekräftige Kenngrößen für die Aktivierung und Desaktivierung eines Enzyms ermittelt werden. Aus den optimierten Parameterwerten werden anschliessend Parameter wie die Halbwertszeit des Biokatalysators oder die Total Turnover Number (TTN, TTN = Mole Produkt/Mol Katalysator) errechnet einschliesslich ihrer Schätzgenauigkeit.

Erst diese Werte, die erfindungsgemäß gefunden werden, sind geeignet, das Langzeitverhalten und die Prozeßstabilität eines Enzyms in einem enzymatisch katalysierten Verfahren in zuverlässiger Form vorherzusagen.

Eventuell ist ein iteratives Vorgehen bei den Schritten 2. bis 4. notwendig, sodass sich erneut ein Optimum Experimental Design und erneut eine Parameterschätzung sowie eine Errechnung der Kenngrössen wie die Halbwertszeit und TTN anschliessen. Bei diesem iterativen Vorgehen ist es möglich, alle zuvor ermittelten experimentellen Daten und vorläufigen Parameterschätzwerte, vor allem bei der Bestimmung neuer, zusätzlicher Temperatur-Zeit-Profile, miteinzubeziehen.

Das erfindungsgemäße Verfahren ist für alle Enzyme geeignet, die sich entsprechend dem oben skizzierten Modell verhalten.

Beispiele

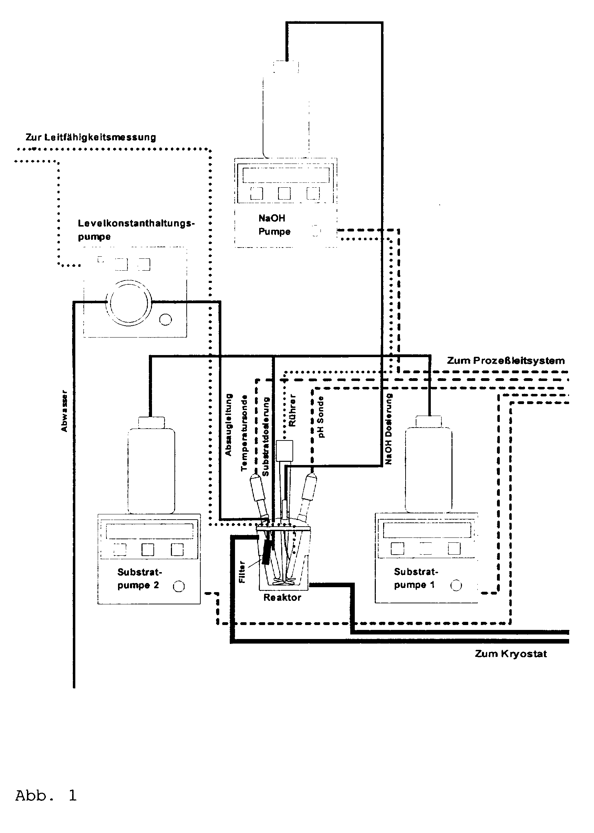

1. Versuchsaufbau:
Der Versuchsaufbau (siehe Abb. 1) besteht aus:

3 Rechnern im Verbund mit einem installiertem Prozeßleitsystem (Fix der Firma Intellution) zur Erfassung und Regelung der Reaktionstemperatur und des pH-Wertes sowie zur Erfassung des dosierten NaOH-Volumens und aller übrigen Versuchsparameter;

2 Dosimat (Typ 665 der Firma Metrohm) für die Substratdosierung (im Wechsel betrieben);

1 Dosimat (Typ 665 der Firma Metrohm) für die Dosierung der 0,1M Natronlauge zur pH-Regelung;

1 Kryostat (Typ RC6CS der Firma Lauda) zur Temperaturregelung;

1 Schlauchpumpe (Typ Mk2 der Firma Watson Marlow).

2. Eingesetzte Substanzen:
Triacetin (Firma Fluka, Best. Nr. 90240) eingesetzt als 0,1M Lösung mit vollentsalztem Wasser verdünnt.
1M NaOH Maßlösung (Firma Merck, Best. Nr.109137).
Eigenfixiertes Enzym Novozym 525 (Firma Novo) auf DAP.

3. Versuchsdurchführung:
Der gereinigte DEO-Reaktor wird mit 50 ml einer 0,1 M Triacetinlösung gefüllt. Anschließend wird der Zulauf von Substrat gestartet (4ml/min) und die Temperatur auf 30°C eingestellt. Nach dem Erreichen der Starttemperatur gibt man das abgewogene Enzym hinzu und startet die pH-Regelung (pH7,0). Bei Erreichen des voreingestellten pH-Wertes werden die zuvor definierten Temperatur-Zeit-Profile gestartet.

4. Datenerfassung:
Die Daten werden mittels Prozeßleitsystem erfasst, gespeichert und in Microsoft Excel eingelesen. Die NaOH-Verbrauchswerte werden in ml/min umgerechnet und davon die für chemische Hydrolyse verbrauchten Mengen (zuvor in einem Temperaturrampenversuch ermittelt ) abgezogen. NaOH-Verbrauchswerte, integrierter Gesamt-NaOH-Verbrauch, Temperatur und pH-Wert werden in entsprechenden Diagrammen gegen der Zeit aufgetragen.

**Beispiel 1**

Vorgehen nach dem Stand der Technik:

Die Parameter wurden auf Basis eines Experimentes mit linearem Temperaturanstieg von 10 °C/h und einer Endtemperatur von 70 °C ermittelt.

Folgende Startwerte wurden für die Parameteridentifikation verwendet:

$$\Delta h_E{}^* \quad = 35 \quad [kJ/mol]$$

$$\ln(A) \quad = 22 \quad \Rightarrow A = 3.58*10^9 \quad [1/min]$$

$$\Delta h_{d,U}{}^* \quad = 123 \quad [kJ/mol]$$

$$\Delta h_{d,N}{}^* \quad = 241 \quad [kJ/mol]$$

$$\ln(\Delta k_{d,U}{}^0) \quad = 43 \quad \Rightarrow \Delta k_{d,U}{}^0 = 4.73*10^{18} \quad [1/min]$$

$$\ln(\Delta k_{d,N}{}^0) \quad = 86 \quad \Rightarrow \Delta k_{d,N}{}^0 = 2.24*10^{37} \quad [1/min]$$

$$\Delta h_U{}^0 \quad = 594 \quad [kJ/mol]$$

$$\Delta s_U{}^0 \quad = 1.8 \quad [kJ/(mol\ K)]$$

Nach Durchführung der Parameteridentifikation ergaben sich folgende Parameterwerte und Schätzgenauigkeiten:

$$\Delta h_E{}^* \quad = 49 \pm 10.9 \quad [kJ/mol]$$

$$\ln(A) \quad = 27.9 \pm 4.42 \quad \ln([1/min])$$

$$\Delta h_{d,U}{}^* \quad = 123.1 \pm 6.2*10^8 \quad [kJ/mol]$$

$$\Delta h_{d,N}{}^* \quad = 90.9 \pm 39 \quad [kJ/mol]$$

$$\ln(\Delta k_{d,U}{}^0) \quad = 43.01 \pm 2.2*10^8 \quad \ln([1/min])$$

$$\ln(\Delta k_{d,N}{}^0) \quad = 28.7 \pm 14.6 \quad \ln([1/min])$$

$$\Delta h_U{}^0 \quad = 634.2 \pm 8.1*10^8 \quad [kJ/mol]$$

$$\Delta s_U{}^0 \quad = 1.7 \pm 2.4*10^5 \quad [kJ/(mol\ K)]$$

Die sich ergebenden Schwankungsbreiten zum Beispiel für $\Delta h_{d,U}{}^*$ mit $\pm$ 6,2 * $10^8$ [kJ/mol] zeigen deutlich, daß diese Schätzungen keine verläßliche Grundlage für ein reaktionskinetisches Prozeßmodell bieten.

Beispiel 2

Durchführung und Auswertung der Versuche:

Für das immobilisierte beispielhaft eingesetzte Enzym *Novozym 525 auf DAP* wurde zunächst ein Temperaturprofil mit einem linearen Temperaturanstieg von 10 °C/Stunde und einer Endtemperatur von 70 °C durchgeführt. Aus dem resultierenden Verlauf der Umsatz-Zeit-Kurve (NaOH-Verbrauch in [ml/min]) wurde ein erster Schätzwert für die Schmelztemperatur bestimmt. Der Schmelzpunkt befindet sich näherungsweise 2 °C oberhalb der Temperatur, an der sich das Maximum der Umsatz-Zeit-Kurve befindet. In diesem Anwendungsbeispiel ergab sich ein Wert von 60 °C für die Schmelztemperatur. Weiterhin wurden im Temperaturbereich von 30 bis 45 °C mit Hilfe eines Arrhenius-Diagramms Schätzwerte für die Parameter $\Delta h_E{}^*$ und A ermittelt. Dies ist zulässig, da die Aktivierungsreaktion in diesem Temperaturbereich dominiert, während die übrigen Reaktionen in erster Näherung vernachlässigt werden können.

Sechs Versuche zur Ermittlung der Anfangsschätzungen für die Desaktivierungsparameter $\Delta h_{d,U}{}^*$, $\Delta h_{d,N}{}^*$, $k_{d,U}{}^0$ und $k_{d,N}{}^0$ wurden analog zum ersten Versuch für die Endtemperaturen 50, 55, 57, 63, 65 und 70 (Wiederholungsversuch) °C durchgeführt. Die Versuche wurden so lange durchgeführt, bis ein Abfall der Enzymaktivität auf ca. 50% der Anfangsaktivität vorlag. Aus diesen Versuchen wurden mit Hilfe gängiger rekursiver Least-Squares-Schätzverfahren die entsprechenden Halbwertszeiten und aus diesen wiederum die resultierenden Werte der Desaktivierung errechnet. Die Auftragung dieser Desaktivierungswerte in einem Arrhenius-Diagramm ermöglicht die Bestimmung der Desaktivierungsparameter $\Delta h_{d,U}{}^*$, $\Delta h_{d,N}{}^*$, $k_{d,U}{}^0$ und $k_{d,N}{}^0$. Die Parameter für den Zerfall des nativen Enzym werden aus diesem Diagramm unterhalb, die für den Zerfall des entfalteten Enzym oberhalb des Schmelzpunktes bestimmt.

Aus dem Schnittpunkt der linearen Approximationen im Arrhenius-Diagramm ($\ln(k_{des})$ vs. 1/T) für die Meßwerte oberhalb bzw. unterhalb des Schmelzpunktes wird eine korrigierte Schätzung für die Schmelztemperatur abgelesen.

Der noch fehlende Parameter für die Beschreibung des Entfaltungsvorganges wird aus dem Residuum der gemessenen Reaktionsrate (Laugenverbrauch in [ml/min]) abzüglich des Aktivierungsanteiles im Temperaturbereich von ca. 45 bis ca. 60 °C aus dem Versuch mit Endtemperatur 70 °C bestimmt. Dieser Bestimmung liegt die Annahme zugrunde, daß in diesem Temperaturbereich neben der Aktivierungsreaktion auch die als sehr schnell angenommene Entfaltungsreaktion zum Tragen kommt, während die langsameren Desaktivierungsreaktionen in diesem schnell durchlaufenen Temperaturbereich in erster Näherung vernachlässigt werden.

Die so bestimmten acht Startwerte für die kinetischen Parameter dienen als Grundlage für die Berechnung neuer Temperatur-Zeit-Profile auf Basis des Verfahrens des Optimum Experimental Designs. Dieses Verfahren berechnet diejenigen Temperatur-Zeit-Profile als Vorgabe für die sich anschließenden weiteren Experimente, die bei gegebenem Versuchsaufwand zu einer Minimierung des zu erwartenden Schätzfehlers für die Parameter führen.

Mit jedem zusätzlich durchgeführten Experiment reduziert sich dabei der zu erwartende Schätzfehler für die Parameter.

Nach der experimentellen Durchführung dieser berechneten Temperatur-Zeit-Profile erfolgt die Durchführung der Parameterschätzung mit Hilfe des Randwertproblemansatz. Es kam dabei das Softwaretool PARFIT des Interdisziplinären Zentrums für wissenschaftliches Rechnen (IWR) der Universität Heidelberg zum Einsatz.

Abschließend werden die auf Basis dieser verbesserten Parameterwerte mit Hilfe eines entsprechenden Simulationsmodells die gewünschten enzymatischen Kenngrößen Total turnover number (TTN) und Halbwertszeit inclusive der zugehörigen Schätzgenauigkeiten berechnet.

Auf Basis der Anfangsschätzwerte (identisch mit den Startwerten oben)

$$\Delta h_E{}^* \quad = 35 \quad [\text{kJ/mol}]$$

$$\ln(A) \quad = 22 \quad \Rightarrow A = 3.58 * 10^9 \quad [\text{1/min}]$$

$$\Delta h_{d,U}{}^* \quad = 123 \quad [\text{kJ/mol}]$$

$$\Delta h_{d,N}{}^* \quad = 241 \quad [\text{kJ/mol}]$$

$$\ln(\Delta k_{d,U}^{0}) \quad = 43 \quad \Rightarrow \Delta k_{d,U}^{0} = 4.73*10^{18}[1/min]$$

$$\ln(\Delta k_{d,N}^{0}) \quad = 86 \quad \Rightarrow \Delta k_{d,N}^{0} = 2.24*10^{37} \quad [1/min]$$

$$\Delta h_{U}^{0} \quad = 594 \quad [kJ/mol]$$

$$\Delta s_{U}^{0} \quad = 1.8 \quad [kJ/(mol\ K)]$$

wurde ein Optimum Experimental Design durchgeführt. Es wurden für dieses Anwendungsbeispiel insgesamt drei Szenarien durchgespielt:

Berechnung eines zusätzlichen Experiments (one experimental design), Berechnung von zwei zusätzlichen Experimenten (two experimental design) und

Berechnung von drei zusätzlichen Experimenten (three experimental design).

Die Parameterschätzung wurde nun unter Berücksichtigung von insgesamt sechs Experimenten durchgeführt (Experimente des two and three experimental design plus des Experimentes mit linearem Temperaturanstieg von 10 °C/h und einer Endtemperatur von 70 °C).

Nach Durchführung der Parameteridentifikation ergaben sich folgende Parameterwerte und Schätzgenauig-keiten:

$$\Delta h_{E}^{*} \quad = 50.2 \pm 2.0 \quad [kJ/mol]$$

$$\ln(A) \quad = 27.8 \pm 0.8 \quad \ln([1/min])$$

$$\Delta h_{d,U}^{*} \quad = 293.7 \pm 388.6 \quad [kJ/mol]$$

$$\Delta h_{d,N}^{*} \quad = 189.8 \pm 32.5 \quad [kJ/mol]$$

$$\ln(\Delta k_{d,U}^{0}) \quad = 98.7 \pm 133.4 \quad \ln([1/min])$$

$$\ln(\Delta k_{d,N}^{0}) \quad = 63.4 \pm 12 \quad \ln([1/min])$$

$$\Delta h_{U}^{0} \quad = 185.3 \pm 29 \quad [kJ/mol]$$

$$\Delta s_{U}^{0} \quad = 0.6 \pm 0.1 \quad [kJ/(mol\ K)]$$

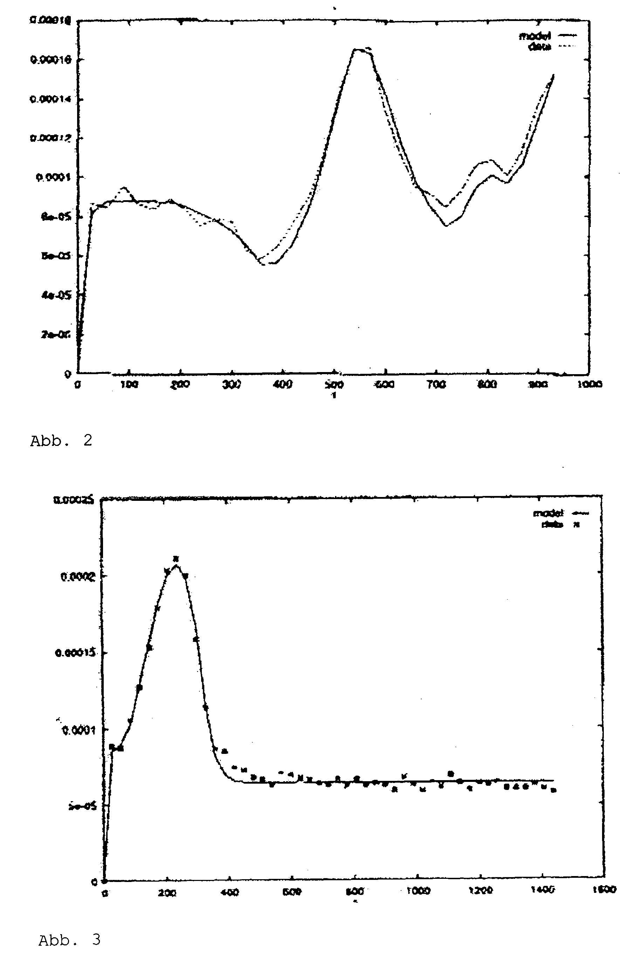

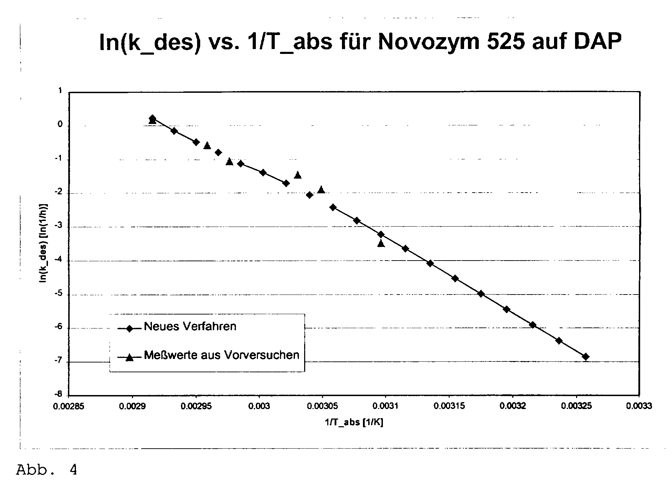

Die sich daraus resultierende Anpassung der Meßdaten ist für zwei exemplarisch ausgewählte Experimente in Abb. 2 und 3 zu sehen. Die Verbesserung der Parameterschätzung schlägt sich in verbesserten Schätzwerten für die Kennzahlen zur Charakterisierung des Langzeitverhaltens der Enzyme nieder (siehe Tabelle 1). Aus Abb. 4 ist weiterhin zu erkennen, daß die aus den Halbwertszeiten aus Tabelle 1 berechneten $\ln(k_{des})$-Werte sehr gut mit den experimentell ermittelten Wertes der sechs Vorversuche übereinstimmen.

Tabelle 1

| Die auf Basis dieser Schätzwerte berechneten Kennzahlen zur Charakterisierung des Langzeitverhaltens der Enzyme ergeben sich wie folgt: | | |
|---|---|---|
| Temperatur [°C] | Halbwertszeit [min] | Total turnover number [mol/mol] |
| 34 | 39529 ± 30160 | $(172.6 \pm 133.4) *10^6$ |
| 36 | 24456 ± 16820 | $(121.2 \pm 81.2) *10^6$ |
| 38 | 15234 ± 9280 | $(85.4 \pm 50.5) *10^6$ |
| 40 | 9555 ± 4988 | $(60.5 \pm 31.3) *10^6$ |
| 42 | 6036 ± 2723 | $(43.0 \pm 18.6) *10^6$ |
| 44 | 3843 ± 1450 | $(30.8 \pm 11.0) *10^6$ |
| 46 | 2468 ± 754 | $(22.1 \pm 6.4) *10^6$ |
| 48 | 1601 ± 383 | $(15.9 \pm 3.5) *10^6$ |
| 50 | 1050 ± 186 | $(11.5 \pm 1.9) *10^6$ |
| 52 | 699 ± 93 | $(8.3 \pm 1.0) *10^6$ |
| 54 | 473 ± 50 | $(6.0 \pm 0.5) *10^6$ |
| 56 | 326 ± 31 | $(4.4 \pm 0.4) *10^6$ |
| 58 | 230 ± 20 | $(3.2 \pm 0.3) *10^6$ |
| 60 | 167 ± 12 | $(2.3 \pm 0.2) *10^6$ |
| 62 | 128 ± 9 | $(1.6 \pm 0.1) *10^6$ |
| 64 | 91 ± 12 | $(1.1 \pm 0.1) *10^6$ |
| 66 | 67 ± 12 | $(0.7 \pm 0.1) *10^6$ |
| 68 | 48 ± 10 | $(0.5 \pm 0.1) *10^6$ |
| 70 | 33 ± 9 | $(0.3 \pm 0.1) *10^6$ |

**Patentansprüche**

1.  Mehrstufiges Verfahren zur Bestimmung der Prozeßstabilität von Enzymen durch Aufnahme von Temperatur-Zeit-Profilen der zu prüfenden Reaktion in einem kontinuierlich betriebenen, instationären Differential-, Kreislauf- oder Rührreaktor und Ermittlung der charakteristischen Aktivitäts- und Stabilitätsparameter durch numerische Methoden in bevorzugt vier Schritten.

2.  Verfahren gemäß Anspruch 1,
    **dadurch gekennzeichnet,**
    daß man folgende Schritte durchführt:

    1. Experimentelle Ermittlung von acht Startparametern und Berechnung der Arrhenius-Kurve der Desaktivierung (ln $k_{des,J}$) mit den Startwerten,

    2. Mathematische Berechnung von speziellen Temperatur-Zeit-Profilen als Vorgabe für neue Experimente zur Aufnahme von Umsatz-Zeit-Kurven.

    3. Auf Basis dieser neuen experimentellen Daten erfolgt die Verbesserung der Parameterschätzung mit Hilfe bekannter numerischer Optimierungtsverfahren und eine Angabe der Schätzgüte für diese Parameter.

    4. Errechnung der prozeßrelevanten Parameter:

        i) total turnover (TTN),
        ii) Halbwertszeit ($\tau_{1/2}$) und

iii) (optional): optimale Temperatur $T_{opt}$ bei bestimmter Mindestraumzeitausbeute ggf. inclusive der Angabe der Schätzgenauigkeiten, mit einem ggf. iterativen Durchlaufen der Schritte 2 bis 4

3. Verfahren gemäß den Ansprüchen 1 oder 2,
**dadurch gekennzeichnet,**
daß man

1.1 in einen Temperaturbereich von ca. 20°C bis 90°C den Reaktionsverlauf mißt und daraus

1.1.1 den Schmelzpunkt $T_m$ und

1.1.2 die Aktivierungsparameter $\Delta h_E^*$ und A bestimmt,

1.2 die Desaktivierungs- bzw. die Halbwertszeit des Enzyms bei $\geq$ sechs Temperaturen ermittelt,

1.2.1 die zugehörigen Enthalpien $\Delta h_{d,U}^*$, oberhalb und $\Delta h_{d,N}^*$ unterhalb $T_m$ aus dem zugehörigen Arrhenius-Diagramm ebenso

1.2.2 die Desaktivierungsentropien des nativen Zustands $k_{d,N}^0$ und des entfalteten Zustands $k_{d,U}^0$ aus den Halbwertszeiten $\tau_{1/2}$ der Desaktivierungskurven unterhalb ($k_{d,N}^0$) und oberhalb ($k_{d,N}^0$) ebenfalls aus dem Arrhenius-Diagramm, und

1.2.3 $\Delta h_U^0$ und $\Delta s_U^0$ durch Extrapolation der Geraden aus dem Arrhenius-Diagramm (2.1.1) und Bestimmung des Schnittpunktes. Der x-Wert entspricht $1/T_m$. $\Delta s_U^0$ wird aus dem Residuum der gesamten Reaktionsrate abzüglich des Aktivierungsanteiles in einem angemessenen Temperaturbereich um den Schmelzpunkt $T_m$ ermittelt. Die Bestimmung der Parameter erfolgt über ein Arrhenius-Diagramm für das Residuum. Dabei gilt $\Delta h_u^0 = T_m \cdot \Delta s_U^0$.
Alternativ oder zusätzlich können zur Bestimmung der Startwerte gemäß den Schritten 1.1 und 1.2 auch gängige Parameterschätzverfahren eingesetzt werden.

4. Verfahren gemäß einem oder mehreren der vorhergehenden Ansprüche,
**dadurch gekennzeichnet,**
daß das Enzym in löslicher oder in einer auf einem Trägermaterial immobilisierten Form eingesetzt wird.

Abb. 1

Abb. 2

Abb. 3

ln(k_des) vs. 1/T_abs für Novozym 525 auf DAP

Abb. 4

Europäisches
Patentamt

**EUROPÄISCHER RECHERCHENBERICHT**

Nummer der Anmeldung

EP 00 71 0008

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X,D | DE 195 46 192 A (H. VOSS) 12. Juni 1997 (1997-06-12) * Anspruch 1; Abbildungen 2,3 * | 1 | C12Q1/00 C12M1/34 |
| A | | 2-4 | |
| | --- | | |
| X | CHEMICAL ABSTRACTS, vol. 131, no. 25, 20. Dezember 1999 (1999-12-20) Columbus, Ohio, US; abstract no. 335853, XP002149131 * Zusammenfassung * & M. BOY ET AL.: "A method for fast determination of biocatalyst process stability" PROCESS BIOCHEMISTRY, Bd. 34, Nr. 6,7, 1. Juni 1999 (1999-06-01), Seiten 535-547, Oxford UK | 1 | |
| A | | 2-4 | |
| | ----- | | |

RECHERCHIERTE SACHGEBIETE (Int.Cl.7)

C12Q
C12M

Der vorliegende Recherchenbericht wurde für alle Patentansprüche erstellt

| Recherchenort | Abschlußdatum der Recherche | Prüfer |
|---|---|---|
| DEN HAAG | 4. Oktober 2000 | Van Bohemen, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

......................................................................

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 00 71 0008

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

04-10-2000

| Im Recherchenbericht angeführtes Patentdokument | Datum der Veröffentlichung | Mitglied(er) der Patentfamilie | Datum der Veröffentlichung |
|---|---|---|---|
| DE 19546192    A | 12-06-1997 | KEINE | |

Für nähere Einzelheiten zu diesem Anhang : siehe Amtsblatt des Europäischen Patentamts, Nr.12/82

EPO FORM P0461